# EUROPÄISCHE PATENTSCHRIFT

(11) **EP 0 383 201 B1**
(45) Veröffentlichungstag und Bekanntmachung des Hinweises auf die Patenterteilung: **03.05.1995**
(21) Anmeldenummer: 90102537.9
(22) Anmeldetag: 09.02.1990
(51) Int. Cl.: C12P 1/02, A01N 63/04, C12N 1/14

(54) **Trichoderma-Pilz und ihn enthaltendes Fungizid**
Trichoderma fungi and fungicide containing same
Champignon de l'espèce trichoderme et fongicide le contenant

(30) Priorität: 16.02.1989 DE 3904710
(43) Veröffentlichungstag der Anmeldung: 22.08.1990
(73) Patentinhaber: BASF Aktiengesellschaft, 67063 Ludwigshafen (DE)
(72) Erfinder: Speakman, John-Bryan, Dr., D-6800 Mannheim 1 (DE); Scherer, Maria, D-6741 Godramstein (DE); Anke, Heidrun, Dr., D-6750 Kaiserslautern (DE)

(56) Entgegenhaltungen:
- EP-A- 0 133 878
- CHEMICAL ABSTRACTS, Band 108, 1988, Seite 200, Zusammenfassung Nr. 89368v, Columbus, Ohio, US; T. BAICU: "Combination of pesticides and the antagonistic fungus Trichoderma viride Pers. for control of plant pathogenic fungi", &AN. INST. CERCET. PROT. PLANT., ACAD. STIINTE AGRIC. SILVICE 1987, 20, 187-202

## Beschreibung

Gegenstand der Erfindung ist der Pilz Trichoderma sp-35/84, ein Fungizid, das ihn enthält und ein Verfahren zur Bekämpfung der Pythium-Fäule, wobei der Pilz Trichoderma sp-35/84 auf die Pflanzensamen (Saatgüter) oder den Erdboden appliziert wird.

Es ist bekannt, daß Pflanzen und ihre Keimlinge während des Keimens und des Auflaufens der Pflanzen durch Pilze, die im Boden vorkommen (bodenbürtige Pilze), befallen und geschädigt werden können.

Es ist bekannt, daß einige Pilze selbst fungizide Wirkung haben können. In Chemical Abstract 108, 89368v (1988) wird die gemeinsame Verwendung eines solchen Pilzes, Trichoderma viride, und Pestiziden zur Bekämpfung von Schadpilzen beschrieben.

Eine wichtige Gruppe von pflanzenschädigenden Bodenpilzen sind die Pythium-Pilze, die Pythium-Fäule hervorrufen.

Bei vorliegen dieser Pilzerkrankung werden die Stengelgründe von Keimlingen weich und sie fallen zusammen, oder der Pilz dringt über die wurzeln in die wasserleitungsbahnen der Pflanze ein und verstopft diese, was zu dem Welken der Pflanze führt. Erhebliche Ausfälle an Pflanzen und Ertragseinbußen können dann die Folge sein.

Die EP-A-0 133 878 lehrt, Pythium-Schadpilze unter Zuhilfenahme des Pilzes Trichoderma harzianum T-315 zu bekämpfen.

Es wurde festgestellt, daß der Pilz Trichoderma sp-35/84 zur Bekämpfung von Pilzen, insbesondere bodenbürtigen Pilzen, speziell Pythium-Pilzen vorteilhaft geeignet ist.

Trichoderma sp-35/84 ist eine natürlich vorkommende Pilzart. Sie ist unter der Nummer IMI 311 756 beim C.A.B. International Mycological Institute, Kew, England und unter der Nummer DSM 5067 bei der Deutschen Sammlung von Mikroorganismen und Zellkulturen, Braunschweig, Bundesrepublik Deutschland, hinterlegt.

Gegenstand der vorliegenden Erfindung ist unter anderem ein Verfahren zur Bekämpfung der Pythium-Fäule bei unterschiedlichen Pflanzenarten, wobei man die Pilzart Trichoderma sp-35/84 auf die Samen appliziert oder dem Boden beimischt. Trichoderma sp-35/84 ist kein Pathogen für Mensch oder Tier und kein pflanzenpathogener Pilz.

Bodenbürtige Pilze sind beispielsweise Pythium-Pilze. Der Pilz Trichoderma wird in fungzider Menge z.B. in einer Aufwandmenge von 10⁶ bis 10¹⁰ koloniebildenden Einheiten je ml Sporensuspension verwendet. Koloniebildende Einheiten sind sowohl die Sporen als auch Mycel-Bruchstücke. Unter den Pilz Trichoderma sp-35/84 sind sowohl sein Mycel als auch seine Sporen zu verstehen. Die fungizide Wirkung von Trichoderma sp-35/84 zeigt sich besonders gut bei Pflanzen, die für die Pythium-Fäule anfällig sind, z.B. Leguminosen (Erbsen, Bohnen), Kreuzblütlern (Raps, Rettich). Nachtschattengewächsen (Tomaten, Kartoffeln).

Die erfindungsgemäß einzusetzende Pilzart kann durch Kultivieren auf geeigneten Substraten, wie Getreidekörnern, Stroh oder anderen Pflanzenmaterialien, oder auch mit Hilfe von üblichen festen Nährmedien, wie Kartoffeldextrose-Agar, Malz- oder Hefeextrakt-Agar, gegebenenfalls auf geeigneten Trägermaterialien, wie Maismehl-Sand-Mischungen oder Kunststoffen oder auf entsprechenden flüssigen Nährmedien ohne Agarzusatz gezüchtet werden.

Gegenstand der vorliegenden Erfindung sind auch Pflanzenschutzmittel, welche Trichoderma sp-35/84 als Myzel und/oder Sporen neben geeigneten Verdünnungsmitteln, wie wasser und/oder Hilfsmitteln, enthalten. Als Hilfsmittel können übliche Zusatzstoffe, wie Haftmittel oder Emulgatoren, eingesetzt werden.

Der Pilz kann hierbei in Form von koloniebildenden Einheiten z.B. als Myzel oder in Form von Sporen (Konidien, Chlamydosporen) oder Myzelbruchstücken oder Myzelkonglomeraten angewendet werden.

Die erfindungsgemäßen Mittel können als Spritzpulver, emulgierbare Konzentrate, versprühbare Lösungen (Dispersionen), Stäubemittel, Beizmittel, Dispersionen, Granulate oder Mikrogranulate in den üblichen Zubereitungen angewendet werden.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und auch bei wasserdispergierbaren (Mikro-)Granulaten mittels Wasser. Staubförmige, granulierte und Beizmittel-Zubereitungen werden vor der Anwendung üblicherweise nicht mehr mit weiteren inerten Stoffen verdünnt. Auch Mischungen oder Mischformulierungen mit anderen fungiziden oder nicht fungiziden Wirkstoffen, wie Insektiziden, Akariziden, Herbiziden, Düngemitteln oder Wachstumsregulatoren, sind gegebenenfalls möglich, wobei teilweise auch synergistische Wirkungssteigerungen erzielt werden können. Nachstehende Beispiele dienen zur Erläuterung.

### Biologische Beispiele

### A) Vorbereitung des Inokulums:

a) Glasschalen, die 2 %igen Malzextrakt-Agar (MEA) enthielten, wurden mit Sporen oder Myzel von Trichoderma sp-35/84 angeimpft. Der beimpfte Agar wurde 8 bis 21 Tage bei 23 bis 25°C mit oder ohne Schwarzlicht (NUV) bebrütet. Anschließend wurde mit Wasser eine Sporensuspension gewonnen, die auf 10⁶ bis 10¹⁰, insbesondere 10⁸ Sporen /ml verdünnt wurde.
b) wie in a) beschrieben, wurde eine wäßrige Sporensuspension von Trichoderma sp-35/84 hergestellt. Danach erfolgte die Beimpfung mit der frischgewonnenen Sporensuspension in 500 ml Glaskolben, die bereits mit sterilisierten, feuchten gequollenen Haferkörnern gefüllt waren. Nach etwa 14 Tagen, nachdem die Körner vom Pilz überwachsen waren und die Sporen sich gebildet hatten, wurden die verpilzten Körner mit Wasser geschüttelt und die so gewonnene Sporensuspension auf 10⁶ bis 10¹⁰ insbesondere 10⁸ Sporen/ml verdünnt.

### B) Gewächshausversuche

### Beispiel B1

Ein Pythium ultimum-Isolat, das von natürlich befallenen Erbsenpflanzen isoliert worden war, wurde auf 2 %igem MEA kultiviert. Danach erfolgte die Beimpfung von sterilisierten, feuchten Maismehl-Quarzsand-Mischungen bzw. Erde mit Pythium ultimum-Myzel-Agar-Stückchen aus dieser Kultur in 500 ml Glaskolben. Nach etwa 14 Tagen, nachdem das Medium vom Pythium-Pilz durchwachsen war, wurde dieses benutzt, um doppelt gedämpfte Erde, bei einem Mischverhältnis 1 g Pilzinokulum auf 1 kg gedämpfte Erde, mit dem Pathogen zu verseuchen. Man nahm danach 500 g verseuchte Erde und mischte eine 14 Tage alte Agarkultur des Pilzes Trichoderma sp-35/84 unter. Diese behandelte Erde wurde in Plastiktöpfe gefüllt. Die vorbereiteten Töpfe wurden mit je 10 Erbsensamen eingesät und in einer Gewächshauskammer bei 20°C aufgestellt. Als unbehandelte Kontrolle diente die gedämpfte Erde, als verseuchte Kontrolle, die mit dem Maismehl-Quarzsand gemischte Erde und eine unverseuchte aber mit Trichoderma sp-35/84 behandelte Erdprobe zur Phytotoxizitätskontrolle. Der Versuch wurde an drei verschiedenen Terminen wiederholt. Als Vergleich wurde statt Trichoderma sp-35/84 ein Trichoderma harzianum-Stamm verwendet. Die Ergebnisse sind in folgender Tabelle festgehalten.

**Tabelle 1**

| Behandlung | Anzahl gesunder Pflanzen in 2 Töpfen | | | Wirkungsgrad % | | |
|---|---|---|---|---|---|---|
| | I | II | III | I | II | III |
| behandelt | 20 | 20 | 20 | - | - | - |
| verseucht | 0 | 0 | 0 | - | - | - |
| verseucht + Trichoderma sp-35/84 | 10 | 10 | 7 | 50 | 50 | 35 |
| verseucht + Trichoderma harzianum | 6 | 6 | 5 | 30 | 30 | 25 |
| Trichoderma sp-35/84 | 20 | 20 | 20 | - | - | - |
| Trichoderma harzianum | 20 | 20 | 20 | - | - | - |

### Beispiel B2

Mit dem gleichen Pythium ultimum-Isolat wie in B1 wurden Versuche durchgeführt, wobei die Erbsen mit einer wäßrigen Trichoderma sp-35/84-Sporensuspension, die nach Verfahren Aa) erhalten worden war, behandelt wurden. Die Samen wurden 24 Stunden lang in die Suspension getaucht und anschließend in die unterschiedlich verseuchten Erdproben ausgesät. Der Versuch wurde ebenfalls bei 20°C in einem Gewächshaus durchgeführt und an drei verschiedenen Terminen wiederholt. Auch der Vergleich mit Trichoderma harzianum-Stamm wurde durchgeführt. Die Ergebnisse sind in folgender Tabelle festgehalten.

**Tabelle 2**

| Behandlung | Anzahl gesunder Pflanzen in 2 Töpfen | | | Wirkungsgrad % | | |
|---|---|---|---|---|---|---|
| | I | II | III | I | II | III |
| unbehandelt | 20 | 20 | 20 | - | - | - |
| verseucht | 0 | 1 | 3 | - | - | - |
| verseucht + Trichoderma sp-35/84 | 16 | 17 | 12 | 80 | 85 | 60 |
| verseucht + Trichoderma harzianum | 7 | 12 | 6 | 35 | 60 | 30 |
| Trichoderma sp-35/84 | 20 | 20 | 20 | - | - | - |
| Trichoderma harzianum | 20 | 20 | 20 | - | - | - |

### C) Freilandversuche

### Beispiel

Auf eine Fläche, wo Vorversuche gezeigt hatten, daß eine natürliche Pythium-Verseuchung vorlag, wurden Erbsensamen, die nach der unter B2 beschriebenen Methode behandelt worden waren, ausgesät. In drei unabhängigen Versuchen wurden folgende Ergebnisse erzielt.

**Tabelle 3**

| | Ergebnis nach .... Tagen | % Befall in der | | Wirkungsgrad % der Behandlung |
|---|---|---|---|---|
| | | Kontrolle unbehandelt | behandelten Probe | |
| Versuch 1 | 26 | 47,3 | 22,0 | 53,5 |
| Versuch 2 | 30 | 68,8 | 30,0 | 56,4 |
| Versuch 3 | 35 | 89,6 | 11,7 | 86,3 |

Wie die werte aus der obigen Tabelle zeigen, wird die Schädigung der Erbsenpflanzen bei Einsatz von Trichoderma sp-35/84 deutlich herabgesetzt.

## Patentansprüche

1. Pilz Trichoderma sp-35/84, hinterlegt unter der Nummer DSM 5067.

2. Fungizid, enthaltend eine fungzid wirksame Menge des Pilzes gemäß Anspruch 1.

3. Verfahren zur Bekämpfung von Pilzen, dadurch gekennzeichnet, daß man eine fungizid wirksame Menge des Pilzes gemäß Anspruch 1 auf die Pilze oder auf die vor Pilzbefall zu schützenden Saatgüter oder den Boden einwirken läßt.

4. Verfahren nach Anspruch 3, dadurch gekennzeichnet daß man bodenbürtige Pilze bekämpft.

5. Verfahren nach Anspruch 3, dadurch gekennzeichnet, daß man Pythium-Pilze bekämpft.

6. Verfahren nach Anspruch 3, dadurch gekennzeichnet, daß man den Pilz gemäß Anspruch 1 in einer Aufwandmenge von 10⁶ bis 10¹⁰ kolonienbildende Einneiten je ml Sporensuspension anwendet.

7. Verfahren nach Anspruch 3, dadurch gekennzeichnet, daß man eine fungizid wirksame Menge an Pilz gemäß Anspruch 1 auf Samen von Pythium-anfälligen Pflanzen anwendet.

8. Mittel zur Bekämpfung der Pythium-Fäule, enthaltend eine fungizid wirksame Menge des Pilzes gemäß Anspruch 1.

9. Verwendung des Pilzes gemäß Anspruch 1 zur Bekämpfung der Pythium-Fäule.

## Claims

1. The fungus Trichoderma sp-35/84, deposited under the number DSM 5067.

2. A fungicide containing a fungicidal amount of the fungus as claimed in claim 1.

3. A method for controlling fungi, wherein a fungicidal amount of the fungus as claimed in claim 1 is allowed to act on the fungi or on the seed to be protected from fungal attack or on the soil.

4. A method as claimed in claim 3, wherein soil-borne fungi are controlled.

5. A method as claimed in claim 3, wherein Pythium fungi are controlled.

6. A method as claimed in claim 3, wherein the fungus as claimed in claim 1 is used in an application rate of from 10⁶ to 10¹⁰ colony-forming units per ml of spore suspension.

7. A method as claimed in claim 3, wherein a fungicidal amount of fungus as claimed in claim 1 is applied to seeds of plants susceptible to Pythium.

8. An agent for controlling pythium rot, containing a fungicidal amount of the fungus as claimed in claim 1.

9. Use of the fungus as claimed in claim 1 for controlling pythium rot.

## Revendications

1. Champignon, Trichloderma sp 35/84, déposé sous le numéro DSM 5067.

2. Fongicide contenant une quantité efficace comme fongicide du champignon selon la revendication 1.

3. Procédé pour lutter contre les champignons, caractérisé par le fait que l'on fait agir une quantité efficace comme fongicide du champignon selon la revendication 1 sur les champignons ou sur les semences ou le sol à protéger contre une attaque par champignons.

4. Procédé selon la revendication 3, caractérisé par le fait que l'on lutte contre les champignons originaires du sol.

5. Procédé selon la revendication 3, caractérisé par le fait que l'on lutte contre les champignons du genre Pythium.

6. Procédé selon la revendication 3, caractérisé par le fait que l'on utilise le champignon selon la revendication 1 en doses de 106 à 1010 unités formant des colonies par ml de suspension de spores.

7. Procédé selon la revendication 3, caractérisé par le fait que l'on applique une quantité efficace comme fongicide du champignon selon la revendication 1 sur des semences de plantes sujettes au Pythium.

8. Moyen de lutte contre la pourriture par Pythium contenant une quantité efficace comme fongicide du champignon selon la revendication 1.

9. Utilisation du champignon selon la revendication 1 pour lutter contre la pourriture par Pythium.
